Europäisches Patentamt

**European Patent Office**

**Office européen des brevets**

(11) Veröffentlichungsnummer: **0 036 495**
**A1**

(12) **EUROPÄISCHE PATENTANMELDUNG**

(21) Anmeldenummer: **81101330.9**

(51) Int. Cl.³: **H 05 G 1/46, A 61 B 6/00**

(22) Anmeldetag: **25.02.81**

(30) Priorität: **19.03.80 DE 3010592**

(43) Veröffentlichungstag der Anmeldung: **30.09.81**
**Patentblatt 81/39**

(84) Benannte Vertragsstaaten: **AT BE CH DE FR GB IT LI LU NL SE**

(71) Anmelder: **Mediprix AG, Hohenrainstrasse 9, CH-6280 Hochdorf (CH)**

(72) Erfinder: **Laupper, Rudolf, Hohenrainstrasse 9, CH-6280 Hochdorf (CH)**

(74) Vertreter: **Riebling, Günter, Dr. Ing. et al, Rennerle 10 Postfach 3160, D-8990 Lindau (DE)**

(54) **Vorrichtung zur Steuerung der Strahlungsintensität einer Röntgenanlage.**

(57) Die Vorrichtung zur Steuerung der Strahlungsintensität einer Röntgenanlage enthält ein Bedienungsfeld (7), mit einzelnen Tasten (4a bis 4f), denen jeweils eine Beschriftung (5) zugeordnet ist. Neben dem Bedienungsfeld (7) ist ein Sichtfeld (15) mit der Darstellung eines menschlichen Skelettes dargestellt, wobei den betreffenden, zu wählenden Körperteilen oder Organen an der betreffenden Stelle des Skelettes im Sichtfeld (15) eine Kontroll-Lampe (16a bis 16f) zugeordnet ist. Durch Blick auf das Sichtfeld (15) kann die Bedienungsperson nun erkennen, ob sie im Bedienungsfeld (7) die richtige Taste gedrückt hat. Die Auswahl der Einstellungen hinsichtlich des Abstands der Röhre vom Aufnahmeschirm, der KV-Zahl der Röntgenröhre und des Milliampere-Sekunden-Produktes erfolgt automatisch, je nachdem, welche Taste im Bedienungsfeld (7) gedrückt wurde.

Vorrichtung zur Steuerung der Strahlungsintensität
einer Röntgenanlage
------------------------------------------------

Gegenstand der Erfindung ist eine Vorrichtung zur
Steuerung der Strahlungsintensität einer Röntgenanlage am Ort der Röntgenaufnahme durch Änderung des
Abstandes zwischen Röntgenröhre und Buckywand, sowie
Steuerung der Spannung und/oder des Milliampere-
Sekunden-Produktes (MSP) der Röntgenröhre.

Es sind sogenannte Belichtungsautomaten bei Röntgenanlagen bekannt, die die durch den zu durchleuchtenden
Körper gehende Strahlungsintensität messen und in Abhängigkeit davon das Milliampere-Sekunden-Produkt der
Röntgenröhre einstellen. Die Spannung (Kilo-Volt-Zahl)
wird in der Regel vorgewählt, je nachdem, welches Organ durchleuchtet wird. Bei einer Brustdurchleuchtung
z.B. ist die KV-Zahl höher,als wenn nur eine Hand
durchleuchtet wird.

Bisher bestand der Nachteil, dass eine optimale Belichtung bei minimaler Strahlenbelastung des Körpers nur
dadurch möglich wurde, wenn die ausgebildete Bedienungskraft die Röntgenanlage bediente; denn es gehörte eine
gewisse Erfahrung dazu, um entsprechend der Konstitution des Patienten und entsprechend des Knochengerüstes
bei einem älteren oder jüngeren Patienten oder ja nachdem, welches Organ zu durchleuchten war, die richtige
Belichtung herauszufinden. In der Mehrzahl der Fälle
wurde eine zu hohe Durchleuchtungsintensität gewählt,
wodurch der Nachteil einer hohen Strahlenbelastung für
den Patienten bestand und ferner die erhaltenen Aufnahmen stark geschwärzt waren.

Zur Herstellung der optimalen Belichtung ist es bekannt, den Abstand der Röhre vom Aufnahmeschirm (Buckywand) zu ändern; man hat weiterhin die Möglichkeit, die KV-Zahl zu ändern oder das Milliampere-Sekunden-Produkt (MSP) zu ändern.

Diese drei voneinander unabhängig zu ändernden Grössen bieten viele Bedienungsschwierigkeiten. Hier ist ein hohes Maß an Ausbildung für die Bedienungsperson erforderlich, um die richtige Durchleuchtungsintensität zu finden.

Die Erfindung hat sich die Aufgabe gestellt, eine Vorrichtung zur Steuerung der Strahlungsintensität einer Röntgenanlage der eingangs genannten Art so weiterzubilden, dass sie auch mit den geringsten medizinischen und bedienungsmässigen Kenntnissen bedient werden kann und immer dafür gesorgt ist, dass eine optimale Belichtung bei minimaler Strahlenbelastung gegeben ist.

Zur Lösung der gestellten Aufgabe ist die Erfindung dadurch gekennzeichnet, dass durch getrennt voneinander in einem Bedienungsfeld angeordnete Wahlschalter folgende Funktonen getrennt voneinander steuerbar sind:

a) Wahlschalter für die verschiedenen, zu durchleuchtenden Organe und Körperteile (Beschriftung) steuert den Abstand zwischen Röntgenröhre und Buckywand und gibt eine Mindest-Strahlungsintensität vor,

b) Wahlschalter ändert abhängig von der Lage des Patienten im Strahlengang das Milliampere-Sekunden-Produkt (MSP) und von der Grösse der Strahlungsintensität einer Laufrasterlade,

c) Wahlschalter als Sicherheitsschalter im Stromkreis der Aufnahmetaste angeordnet, ändert das MSP bei Abweichung von der körperlichen Norm des Patienten.

Durch die gegebene technische Lehre wird erreicht, dass bei optimalerer Belichtung der Patient nur einer minimalen Strahlungsbelastung ausgesetzt wird. Durch die erfindungsgemässe Zuordnung der Wahlschalter zu den den Wahlschaltern zusteuernden Funktionen wird ein vollkommen neuer Weg bei der Verbesserung der Aufnahmequalität und bei der Verringerung der Strahlungsintensität bei der Durchleuchtung bei gleichzeitiger optimaler Bildwiedergabe erreicht.

Der Wahlschalter, mit dem die verschiedenen zu durchleuchtenden Organe und Körperteile auszuwählen sind, ordnet z.B. bei einer Lungenaufnahme eine höhere Strahlungsintensität, das heisst, ein höheres MSP zu, als wenn nur eine Hand durchleuchtet wird. Gleichzeitig wird auch die KV-Zahl geändert. Die Zuordnung erfolgt aber hier im wesentlichen durch die Steuerung des Abstandes der Röntgenröhre von der Buckywand, da hier quadratisch die Intensität mit abnehmendem Abstand zunimmt.

Bei der Verwirklichung der gegebenen technischen Lehre ist es gleichgültig, in welcher Reihenfolge die eingangs genannten drei Wahlschalter betätigt werden; wesentlich ist immer, dass, wenn einer der Wahlschalter nicht betätigt wird, dann die Auslösung zur Anfertigung der Röntgenaufnahme nicht erfolgt, so dass der Patient keinen unnötigen oder zu hohen Strahlungsbelastungen ausgesetzt wird und hierdurch eine Sicherheit für das Geingen der Röntgenaufnahme gegeben ist.

- 4 -

Der Wahlschalter zur Wahl der Lage des Patienten erhöht
oder verringert das MSP, entsprechend der Lage des Patienten. Insbesondere wird eine Laufrasterlade, welche
zur Verhinderung von Streustrahlen dient, eingeschaltet, wenn ein Mindestmaß oder wenn ein Höchstmaß an
Strahlungsintensität überschritten wird.

Der dritte Wahlschalter ist als Sicherheitsschalter im
Stromkreis der Aufnahmetaste angeordnet und ändert das
MSP bei Abweichung von der körperlichen Norm des Patienten. Durch diese Sicherheitstasten wird eine Feingraduierung in positiver oder negativer Weise vorgegeben,
mit denen die jetzt schon eingestellte Strahlungsintensität vergrössert oder verkleinertwird, je nachdem, ob
es sich um Abweichungen von der körperlichen Norm in positiver oder negativer Hinsicht handelt. Bei einem besonders dünnen oder mageren Patienten wird die Strahlungsintensität herabgesetzt, bei einem fettleibigen Patienten wird sie erhöht. Sie wird ferner erhöht, wenn die
zu durchleuchtenden Knochen sehr kalkhaltig sind, so
dass hier grössere Strahlungsintensitäten erforderlich
sind, um eine einwandfreie Röntgenaufnahme zu gewährleisten.

Wesentlich ist, dass durch diese ganz einfache Maßnahme und Zuordnung von Wahlschaltern und entsprechenden
Funktionen es erreicht wird, dass nun eine viel einfachere und sichere Bedienung möglich ist.

Zur Verbesserung der Bedienungssicherheit dient es auch,
wenn dem Bedienungsfeld mit dem Wahlschalter für die
verschiedenen zu durchleuchtenden Organe und Körperteile ein Sichtfeld mit Darstellung des menschlichen Körpers zugeordnet ist und jedem der Beschriftung entsprechendem Körperteil im Bedienungsfeld für den Wahlschalter eine Kontrollampe an dem betreffenden Körperteil im
Sichtfeld zugeordnet ist.

Hierdurch wählt die Bedienungsperson in dem Bedienungsfeld des Wahlschalters ein bestimmtes Körperteil an
Hand der Beschriftung aus. Damit sich keine Fehler einschleichen können, ist diesem Bedienungsfeld des Wahlschalters ein Sichtfeld zugeordnet, in dem der menschliche Körper als Skelett dargestellt ist. Entsprechend
dem gewählten Körperteil oder Organ aus dem Bedienungsfeld des Wahlschalters leuchtet dann in dem Sichtfeld
an dem betreffenden Teil des Skelettes eine Kontroll-
lampe auf, damit die Bedienungsperson die Sicherheit
hat, auch das richtige Körperteil gewählt zu haben.

Zusätzlich können noch die Tasten im BEdienungsfeld
des Wahlschalters mit Kontrollampen versehen sein, damit nach dem Drücken der Kontrollampen festgehalten
wird, welche der Tasten gedrückt wurde.

In gleicher Weise ist der Wahlschalter zur Wahl der
Lage des Patienten als mit separaten Tasten ausgestattetes Bedienungsfeld ausgebildet, wobei jede Taste
mit einer Beschriftung und gegebenenfalls mit einer
Kontrollampe versehen ist. Das gleiche gilt auch für
den Wahlschalter zur Korrektur bei Abweichungen von
der körperlichen Norm, hier ist wiederum ein mit separaten Tasten ausgestattetes Bedienungsfeld vorgesehen,
bei dem jedem möglichen Korrekturwert eine Taste mit
Beschriftung und gegebenenfalls Kontrollampe zugeordnet ist.

Selbstverständlich brauchen die oben aufgezählten drei
Wahlschalter nicht als in einzelne Tasten aufgelöste

- 6 -

Bedienungsfelder ausgebildet zu sein, es ist in einer
anderen, nicht näher dargestellten Ausführungsform
auch möglich, den jeweiligen Wahlschalter als Drehschalter, Kippschalter vorzusehen.

In einer Weiterbildung der vorliegenden Erfindung ist es
auch vorgesehen, dass die Wahlschalter entfallen und
stattdessen eine Programmierung der Röntgenanlage durch
Lochkarten, Magnetband oder andere Datenträger erfolgt.
Die entsprechenden Schaltzustände, die für den jeweiligen Anwendungsfall gefordert sind, sind dann in digitaler Form in den Datenträger eingespeichert und lösen nach Einspeicherung des Datenträgers in die automatische Datenverarbeitungsanlage der Röntgenanlage
die entsprechenden elektrischen Schaltzustände in
der Steuerung aus.

Wesentlich ist ferner, dass der Wahlschalter zur Wahl
der Durchleuchtung verschiedener Organe und Körperteile
und der Wahlschalter zur Wahl der Lage des Patienten die
Antriebsmotoren des Stativs der Röntgenanlage steuern.
Das Stativ besteht hierbei aus einer auf der Aufstellungsebene angeordneten vertikalen Wandsäule, an der
in vertikaler Richtung verfahrbar ein Vertikalwagen angeordnet ist, an dem schwenkbar ein zweiarmiger Querbalken ansetzt, an dessen einem freien Ende die Buckywand verfahrbar an einem ersten Querwagen und an dessen anderem freien Ende die Röntgenröhre verfahrbar an
einem zweiten Querwagen angeordnet sind.

Die folgenden Bewegungen in einer Ebene können ab Hand
- und integrierter Programmsteuerung ausgeführt werden:
- Vertikalwagen AUF und AB
- Querbalken     AUF und AB sowie DREHEN links und rechts
- Querwagen      links und rechts auf Distanzen von 1 -

-7-

1,2 - 1,5 und 2m
- Halterung für DREHEN links und rechts, um die Aufnahmewinkel  Buckywand zu verändern
- Halterung für AUF und AB im rechten Winkel zum Querwagen, um Röhre Strahlmittelpunkt zu
              verschieben.

Die Bewegungen können einzeln oder in Kombination zueinander ausgeführt werden. Sämtliche Antriebselemente
sind so abgesichert, dass weder Patient, Bedienungspersonal noch Stativ Schaden erleiden kann. Die durch
Eintasten oder durch integrierte Programme erteilten
Signale für die Ausführung der gewünschten Bewegungen
können über Draht oder mit Infrarot- resp. Ultraschallwellen übermittelt werden. Alle Bewegungen können durch
kurzes Signal eingeleitet werden, wobei der Anlauf
zum gewünschten Punkt immer über den kürzesten Weg erfolgt und beim Erreichen automatisch unterbrochen wird.
Bei den Drehbewegungen des Querbalkens sind logisch
verknüpfte Sicherungen vorhanden, welche ein Beschädigen der Anlage am Boden oder an der Decke verhindern. Im weiteren behält die Halterung für die Buckywand bei eingetasteter Tischhöhe ihre Lage ab Boden
immer bei, auch wenn mit dem Höhen- oder dem Querwagen Bewegungen ausgeführt werden.

Das Stativ ist mit folgenden Stellantrieben versehen:
- symmetrische Horizontalverschiebung von Röhre und
  Buckywand
  Die Verschiebung von Buckywand und Röhre kann auf
  die Fixpunkte 1,0 m - 1,2 m - 1,5 m - 2,0 m erfolgen.  Durch einen kurzen Tastendruck auf die entsprechend beschriftete Taste setzt sich die Buckywand und die Röhre symmetrisch zueinander in Bewegung, bis die gewünschte Distanz erreicht wird, und stellt dort automatisch ab. Die Steuerung für die Horizontalverschiebung ist elektrisch gegen Doppelbetätigung abgesichert. Die Horizontalbewegung dieser beiden Wagen ist auf vier Grössen vorprogrammiert. Eine Verschiebung auf andere Distanzen ist möglich.

- Vertikalverschiebung des Horizontalarmes
  Die Vertikalverschiebung erfolgt über je eine Drucktaste für nach oben oder unten. Zugleich ist eine automatische Einmittung des Wagens in der Höhe eingebaut. Um bei senkrechten Aufnahmen die Tischhöhe automatisch zu erreichen, ist eine dritte Taste "Tischhöhe" vorhanden. Ist diese Taste gedrückt, pendelt der Vertikalwagen beim Verstellen der Aufnahmedistanz automatisch wieder in die richtige Tischhöhe. Die Tasten sind auch bei diesem Antrieb elektrisch gegeneinander verriegelt. Am oberen und unteren Ende sind Endabsicherungen eingebaut.

- Drehbewegung des Horizontalarmes
  Der Horizontalarm kann über je eine Taste stufenlos nach links oder rechts gedreht werden, und zwar bis zu + 95° und - 30°. Am Ende sind ebenfalls Endabsicherungen eingebaut. Über zwei weitere Drucktaster kann der Horizontalarm auf zwei vorprogrammierte Stellungen dirigiert werden, im Normalfall 90° und 0°. Wird eine dieser Tasten kurz betätigt, wird automatisch der folgende Ablauf eingeleitet:
  - Horizontalwagen fährt auf Stellung 1,0 m. Vertikalwagen fährt auf Mittelstellung. Die beiden Bewegungen werden gleichzeitig ausgeführt. Sobald diese beiden Stellungen erreicht sind, beginnt automatisch die Drehbewegung bis zum gewünschten

- 9 -

Punkt. Ist dieser Punkt erreicht, kann der Vertikalwagen und der Horizontalwagen wieder verstellt
werden.

- Stellantrieb zu Buckywand
  Durch je eine Taste kann die Buckywand auf $\pm$ 15$^\circ$ geneigt werden, und zwar stufenlos. Mit einer dritten
  Taste kann die Buckywand automatisch wieder in die
  senkrechte Stellung dirigiert werden, wo diese dann
  automatisch abstellt.

- Stellantrieb zu Röntgenröhre
  Durch je eine Taste kann die Röntgenröhre in ihrer
  Höhe zur Buckywand verstellt werden bis $\pm$ 150 mm.
  Durch eine dritte Taste kann die Röhre wieder automatisch in die richtige Höhe (O-Punkt) dirigiert werden.

Alle Logikelemente sind gegen Fehlschaltung verriegelt.
Alle Antriebe sind selbsthemmend und gegen Überlast abgesichert. Die Spannung beträgt 380 Volt 50 Hz. Andere
Spannungen möglich.

Antrieb zum Drehen des Querbalken

---

Der Antrieb wird über total vier Impulstasten gesteuert.
Diese teilen sich auf wie folgt:
- stufenloses Drehen, bis 95$^\circ$ nach links. Dreht, solange
  gedrückt wird. Bei 95$^\circ$ wird durch Endabsicherung unterbrochen.
- stufenloses Drehen, bis 5$^\circ$ nach rechts. Dreht, solange
  gedrückt wird. Bei 5$^\circ$ wird durch Endabsicherung unterbrochen.
- automatisches Drehen durch kurzes Antippen des Tasters 90$^\circ$. Der Antrieb dreht sich automatisch bis zur

-10-

Position 90$^O$ und stellt dort ab. Bevor sich der Drehantrieb in Bewegung setzen kann, müssen die folgenden Bedingungen erfüllt sein:

a) die Querwagen müssen auf der Position 1, 0 m stehen

b) der Vertikalwagen muss in der Mitte stehen

- automatisches Drehen durch kurzes Antippen des Tasters 0$^O$. Der Antrieb dreht sich automatisch bis zur
  Position 0$^O$ und stellt dort ab. Auch hier müssen zuerst die oben genannten Bedingungen erfüllt sein.
  Das Abstellen des Antriebs zum Drehen erfolgt in der
  jeweiligen Position durch einen Endschalter.

Alle vier Impulstasten sind elektrisch oder mechanisch
gegeneinander verriegelt.

Antrieb zu Vertikalwagen

---

Der Antrieb wird über total drei Impulstasten gesteuert.
Eine davon ist mit Rasterung. Die Tasten teilen sich
wie folgt auf:

- stufenloses Heben des Wagens. Fährt, solange Taste
  gedrückt wird. In der obersten Stellung wird der Antrieb durch eine Endabsicherung unterbrochen.

- stufenloses Senken des Wagens. Fährt, solange Taste
  gedrückt wird. In der untersten Stellung wird der Antrieb durch eine Endabsicherung unterbrochen.

- Beim Drücken der Taste "Tischhöhe" bleibt diese eingerastet und setzt die beiden Tasten "AUF/AB" ausser
  Funktion. Hierbei fährt der Vertikalwagen auf eine
  durch einen Endschalter vorbestimmte Höhe. Wird nun
  der Querwagen auf eine andere Position gebracht, zieht
  der Vertikalwagen automatisch nach, bis wieder die
  richtige Höhe erreicht wird. Die Abtastung für die richtige Höhe erfolgt am linken Querwagen. Die Taste
"Tischhöhe" funktioniert nur, wenn der Querwagen
senkrecht steht.
- Um die Bedingungen für das Drehen zu erfüllen, muss
am Vertikalwagen eine Abtastung der Mittelstellung
vorhanden sein. Wird eine Taste für das automatische
Drehen des Querbalkens gedrückt, muss sich der Vertikalwagen in Bewegung setzen, bis er die Mittelstellung erreicht hat.

Antrieb zu Querwagen
_____

Die beiden Querwagen sind durch Drahtseile so verbunden,
dass sie sich symmetrisch zueinander bewegen. Für die
beiden Querwagen sind total vier festeingestellte Positionen vorgesehen, und zwar 1,0 / 1,2 / 1,5 / und 2,0 m.
Für jede Position ist ein Impulstaster vorgesehen. Durch
Antippen des entsprechenden Tasters setzen sich die beiden Querwagen in Bewegung, bis die gewünschte Stellung
erreicht ist, und stellen dort ab. Sobald die beiden Wagen die gewünschte Stellung erreicht haben, wird dies
durch eine Lampe signalisiert. Die beiden Wagen müssen
sich auf dem kürzesten Weg zu der gewünschten Position
hin bewegen. Alle vier Tasten sind elektrisch oder
mechanisch gegeneinander verriegelt.
Wird eine Taste für das automatische Drehen des Querbalkens gedrückt, müssen sich die beiden Querwagen in
Bewegung setzen, bis die Position 1,0 m erreicht ist.

Antrieb zu Buckywand
_____

Die Buckywand kann durch drei Impulstasten gesteuert
werden. Diese teilen sich wie folgt auf:

- stufenloses Heben der Buckywand. Die Buckywand hebt sich, solange gedrückt wird. In der obersten Stellung wird durch eine Endabsicherung der Antrieb unterbrochen.
- stufenloses Senken der Buckywand. Die Buckywand senkt sich, solange gedrückt wird. In der untersten Stellung wird durch eine Endabsicherung der Antrieb unterbrochen. .
- Wird die dritte Taste kurz angetippt, bewegt sich die Buckywand automatisch in die Mittelstellung. Eventuell kann hier eine Taste mit Rasterung verwendet werden.

Antrieb zu Röntgenröhre

Der Antrieb der Röntgenröhre erfolgt in der gleichen Weise wie der Antrieb der Buckywand.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Im folgenden wird die Erfindung an Hand von lediglich einem Ausführungsweg darstellenden Zeichnung näher erläutert. Hierbei gehen aus den Zeichnungen und ihrer Beschreibung weitere erfindungswesentliche Merkmale und Vorteile der Erfindung hervor.

Es zeigen:

Fig. 1 Draufsicht auf das Bedienungsfeld für den Wahlschalter zur Wahl der verschiedenen zu durchleuchtenden Organe und Körperteile

0036495

Fig. 2 Draufsicht auf das Sichtfeld, das dem Bedienungsfeld nach Fig. 1 zugeordnet ist.

Fig. 3 Draufsicht auf die Bedienungsfelder für die
Wahlschalter zur Wahl der Lage des Patienten
und zur Korrektur bei Abweichungen von der körperlichen Norm, sowie auf das Bedienungsfeld
für die funktionelle Steuerung der Röntgenanlage

Fig. 4 Flussdiagramm über den Funktionsablauf bei der
elektrischen Steuerung der Röntgenanlage

Fig. 5 schematisiert die Seitenansicht des Stativs

Fig. 6 perspektivische Ansicht des Stativs in einer anderen Einstellungslage im Vergleich zu Fig. 5.

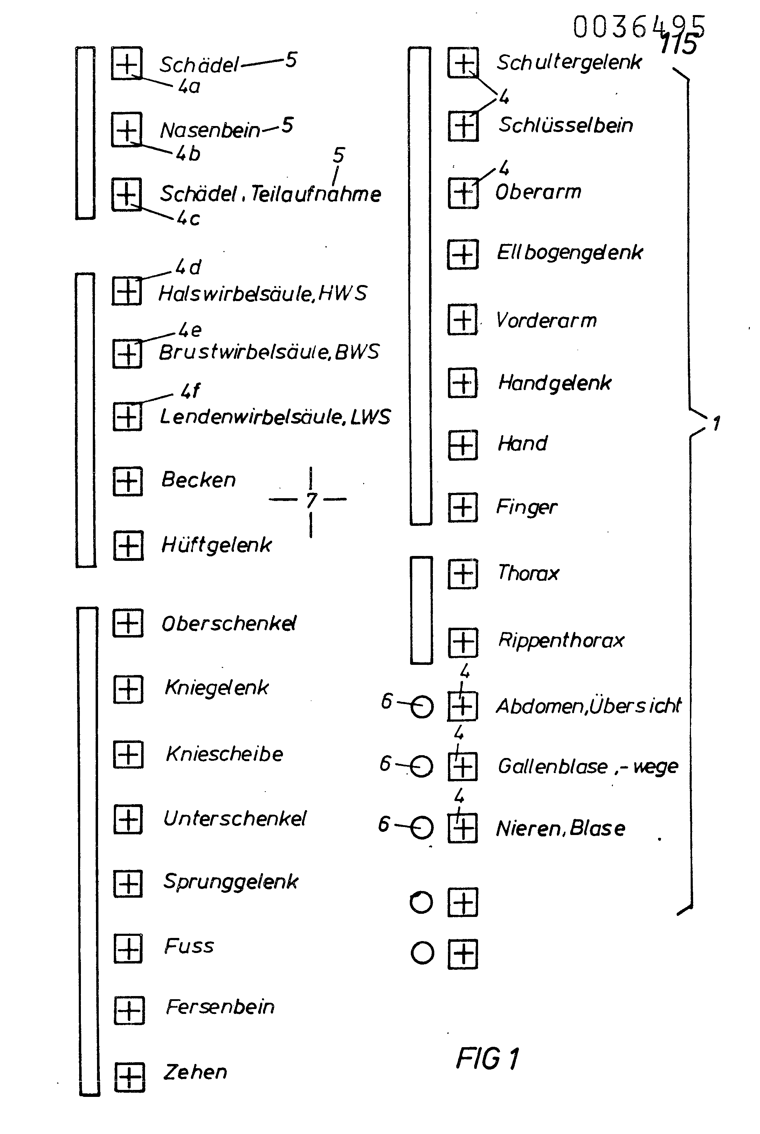

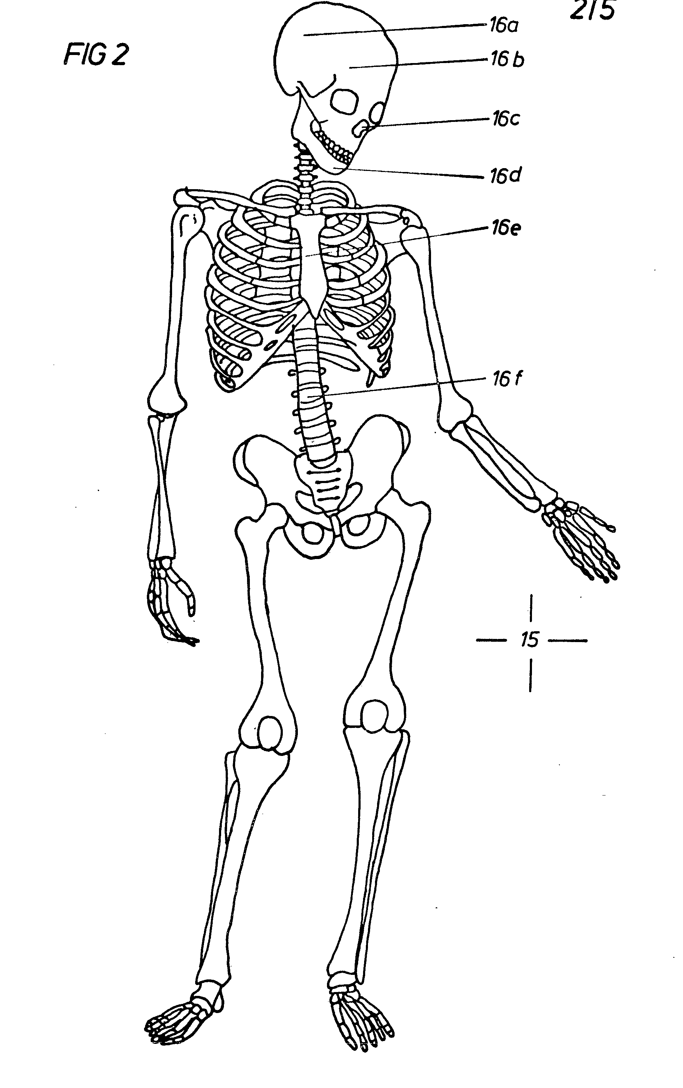

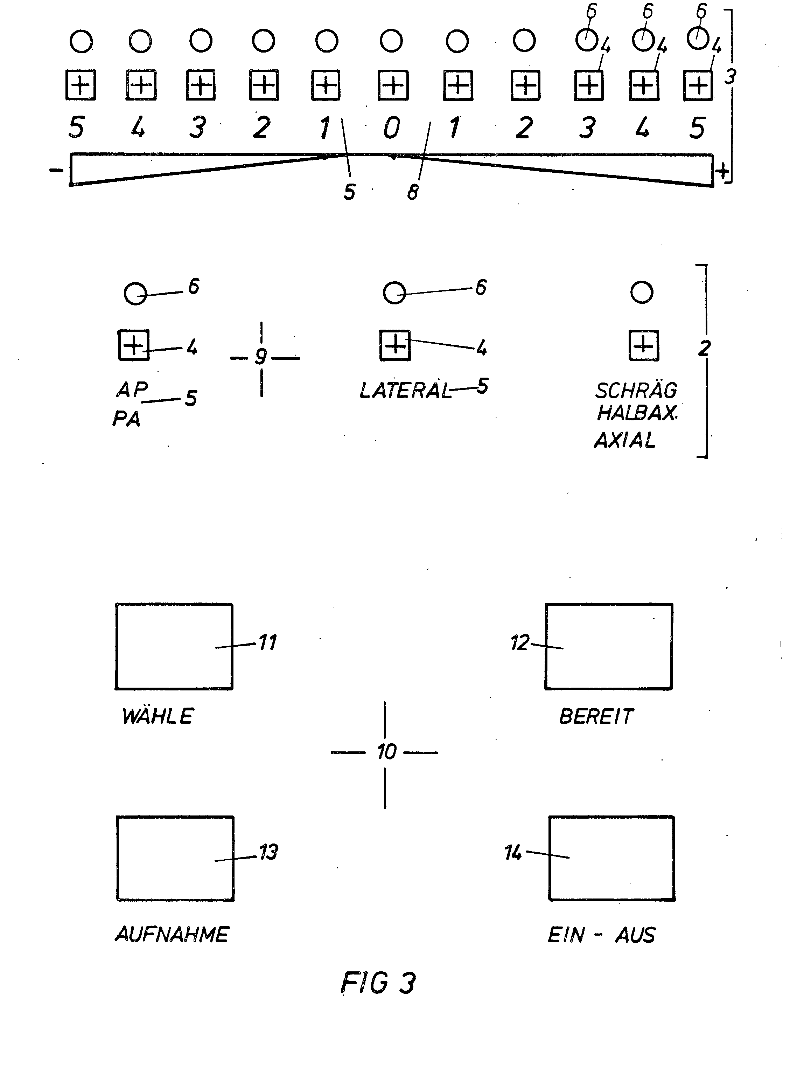

Bezüglich der Fig. 1, 2 und 3 sei vorausgeschickt, dass
die dort gezeigten Bedienungs- bzw. Sichtfelder Taktteil eines insgesamt durchgehenden Sicht- und Bedienungsfeldes sind. Das Sichtfeld 15 nach Fig. 2 ist beispielsweise ganz links auf der Bedienungsfläche der Röntgenanlage angeordnet, daran schliesst sich rechts das
Bedienungsfeld 7 der Fig. 1 an und hieran schliessen
sich wieder rechts die in der Fig. 3 gezeigten Bedienungsfelder 8, 9, 10 an.

Das Bedienungsfeld 7 der Fig. 1 besteht aus einzelnen
Tasten 4a bis 4f, in denen jweils eine Beschriftung 5
zugeordnet ist. Der Taste 4a ist beispielsweise die
Beschriftung "Schädel" zugeordnet, der Taste 4b die
Beschriftung "Schädel, Teilaufnahme" und der Taste 4c
die Beschriftung 5 "Nasenbein".

Bezüglich dieser Tasten 4a - 4f und dazu geordneten Beschriftungen 5 ist im links daneben angeordneten Sichtfeld 15 ein menschliches Skelett dargestellt, wobei den
betreffenden zu wählenden Körperteilen oder Organen an
der betreffenden Stelle des Skelettes im Sichtfeld 15
eine Kontrollampe 16a - 16f zugeordnet ist. Hierdurch
erhält die Bedienungsperson doppelte Sicherheit, denn
zum einen können die Tasten 4a - 4f im Bedienungsfeld 7
beleuchtet sein (vergleiche Kontrollampen 6) und zum
anderen sind der entsprechend beschrifteten Taste 4a -
4f Kontrollampen 16a - 16f in dem Sichtfeld 15 zugeordnet.

Die Bedienungsperson kann nun mit einem Blick auf das
Sichtfeld 15 erkennen, ob sie auch die richtige Taste
gedrückt hat, und gegebenenfalls eine Korrektur vornehmen.

Rechts neben dem Bedienungsfeld 7 für die Tasten 4a -
4f des Wahlschalters 1 sind drei weitere Bedienungsfelder 8, 9, 10 für die Wahlschalter 2, 3 vorgesehen.
Der Wahlschalter 2 mit dem Bedienungsfeld 9 dient zur
Wahl der Lage des Patienten bzw. zur Korrektur der
röntgenröhrenspezifischen Werte entsprechend der gewählten Lage des Patienten.

Durch Drücken der mittleren Taste 4 mit der Beschriftung "lateral" wird eine laterale Röntgenaufnahme angefertigt, während bei Drücken der Taste rechts im
Tastenfeld 9 beim Wahlschalter 2 eine schräge, halbaxiale oder axiale Röntgenaufnahme angefertigt wird.

Der Wahlschalter 3 dient zur Korrektur der röntgenröhrenspezifischen Werte entsprechend der körperlichen
Konstruktion in Abweichung von der körperlichen Norm des Patienten. Den Werten O bis plus 5 und O bis minus 5
sind entsprechende Tasten 4 zugeordnet, wobei jeder
Taste 4 eine Kontrollampe 6 zugeordnet ist, um später
nach dem Drücken überprüfen zu können, welche Taste 4
gedrückt wurde.

Unterhalb der Bedienungsfelder 8, 9 für die Wahlschalter
2, 3 ist ein weiteres Tastenfeld 10 angeordnet. Es ist
eine EIN-AUS-Taste 14 vorgesehen, mit der die gesamte
Anlage ein- oder ausgeschaltet wird.

Nach dem Einschalten über die EIN-AUS-Taste 14 leuchtet
nach einer gewissen Aufheizzeit die Bereitschaftstaste
12 auf. Es muss dann die Wähltaste 11 gedrückt werden,
bevor die Wahlschalter 1, 2, 3 in den Bedienungsfeldern
7, 8, 9 angewählt werden können. Nach der Wahl in den
entsprechenden Bedienungsfeldern 7, 8, 9 mit den
Wahlschaltern 1, 2, 3 kann die Aufnahmetaste 13 gedrückt werden, wodurch die Aufnahme angefertigt wird.

Aus dieser Beschreibung wird deutlich, dass praktisch
ohne Vorbildung der Bedienungsperson eine Röntgenaufnahme ausserordentlich hoher Qualität mit geringer
Strahlenbelastung für den Patienten gewährleistet ist.

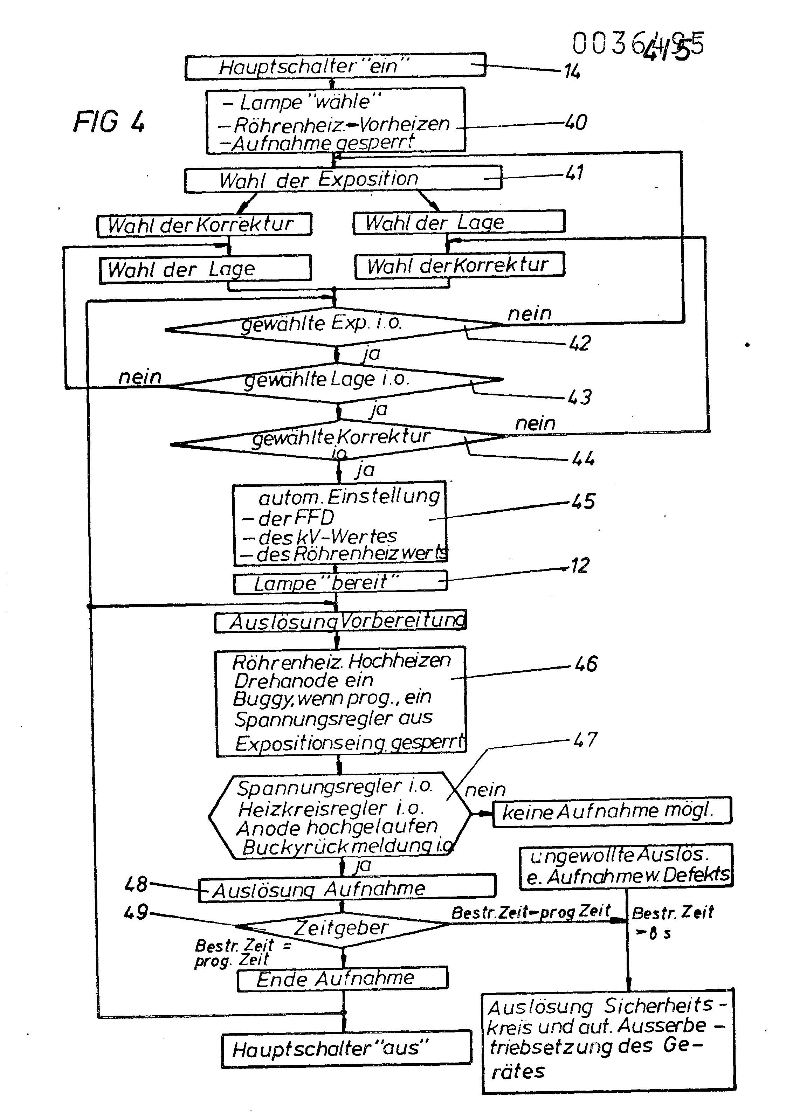

In Fig. 4 ist das Flussdiagramm der elektrischen
Steuerung gezeichnet, wo beginnend von oben in Richtung der eingezeichneten Pfeile zunächst der Hauptschalter mit der EIN-AUS-Taste 14 betätigt wird.
Gemäss dem Funktionsfeld 40 leuchtet dann die Wähltaste 11 auf, die Röhrenheizung für die Röntgenröhre
wird auf "Vorheizen" geschaltet und die Aufnahmetaste
13 wird gesperrt, damit nicht hier bereits schon eine Aufnahme ausgelöst werden kann. Mit dem Funktionsfeld
41 wird dann die Wahl der Exposition entsprechend mit
den Wahlschaltern 1, 2, 3 getroffen. Nach der gewählten
Exposition wird diese Stellung in dem Entscheidungsfeld 42 überprüft. Sollte ein Fehler auftreten, dann
wird die Steuerung wieder in den Funktionszustand des
Funktionsfeldes 41 versetzt, und es muss eine neue Wahl
erfolgen. Ist die gewählte Exposition richtig gewählt
worden (Plausibilitätsprüfung), wird im Entscheidungsfeld 43 geprüft, ob die gewählte Lage in Ordnung ist
oder nicht. Entsprechend der Entscheidung wird entweder
auf das Funktionsfeld 41 zurückgeschaltet oder es wird
weiter geschaltet, um in dem nächsten Entscheidungsfeld 44 eine Überprüfung der gewählten Korrektur (Wahlschalter 3) zu gestatten. Wenn die Stellung der drei
Wahlschalter 1, 2, 3 mit den Entscheidungsfeldern 42,
43, 44 richtig überprüft wurde, erfolgt in dem Funktionsfeld 45 die automatische Einstellung der FFD, des
KV-Wertes und des Röhrenheizwertes. Es leuchtet dann
die Bereitschaftstaste 12 "bereit" auf. Danach wird in
dem nachgeschalteten Funktionsfeld die Röntgenröhre auf
die Aufnahme vorbereitet. Dies erfolgt in dem Funktionsfeld 46 durch Hochheizen der Röhrenheizung durch Einschalten der Drehanode,durch Einschalten des Bucky,
durch Ausschalten des Spannungsreglers und durch Sperren des Expositionseinganges. Mit Durchlaufen dieses
Funktionsfeldes 46 können also die einmal gewählten
Einstellungen an den Wahlschaltern 1, 2, 3 nicht mehr
unbeabsichtigt verändert werden.

Im Entscheidungsfeld 47 wird nun überprüft, ob die
Einstellung des Spannungsreglers in Ordnung ist, ob
der Heizkreisregler in Ordnung ist, ob die Drehanode
hochgelaufen ist und ob die Buckyrückmeldung erfolgt
ist. Bei negativen Entscheidungen ist keine Aufnahme möglich, bei einer positiven Entscheidung wird mit dem Funktionsfeld 48 die Aufnahme ausgelöst.

Es wird nun der Zeitgeber mit dem Entscheidungsfeld 49 eingeschaltet und geprüft, ob die Bestrahlungszeit grösser ist als die programmierte Zeit. Sollte dies der Fall sein oder liegt eine ungewollte Auslösung einer Aufnahme wegen eines Defektes vor, dann erfolgt sofort die Auslösung eines Sicherheitskreises und die automatische Ausserbetriebsetzung des Gerätes. Ebenso erfolgt dieser Vorgang, wenn die Bestrahlungszeit grösser als 8 Sekunden ist. Entspricht die Bestrahlungszeit der programmierten Zeit, dann wird die Röntgenröhre nach der vorgegebenen Zeit abgeschaltet und die Aufnahme ist beendet.

Der Hauptschalter (EIN-AUS-Taste 14) kann danach ausgeschaltet werden oder es kann eine neue Aufnahme durchgeführt werden. Entweder kann hierbei eine neue Lage gewählt werden (einsetzen beim Entscheidungsfeld 42), oder es kann mit der gleichen gewählten Lage eine weitere Aufnahme durchgeführt werden (einsetzen bei der Auslösung "Vorbereitung").

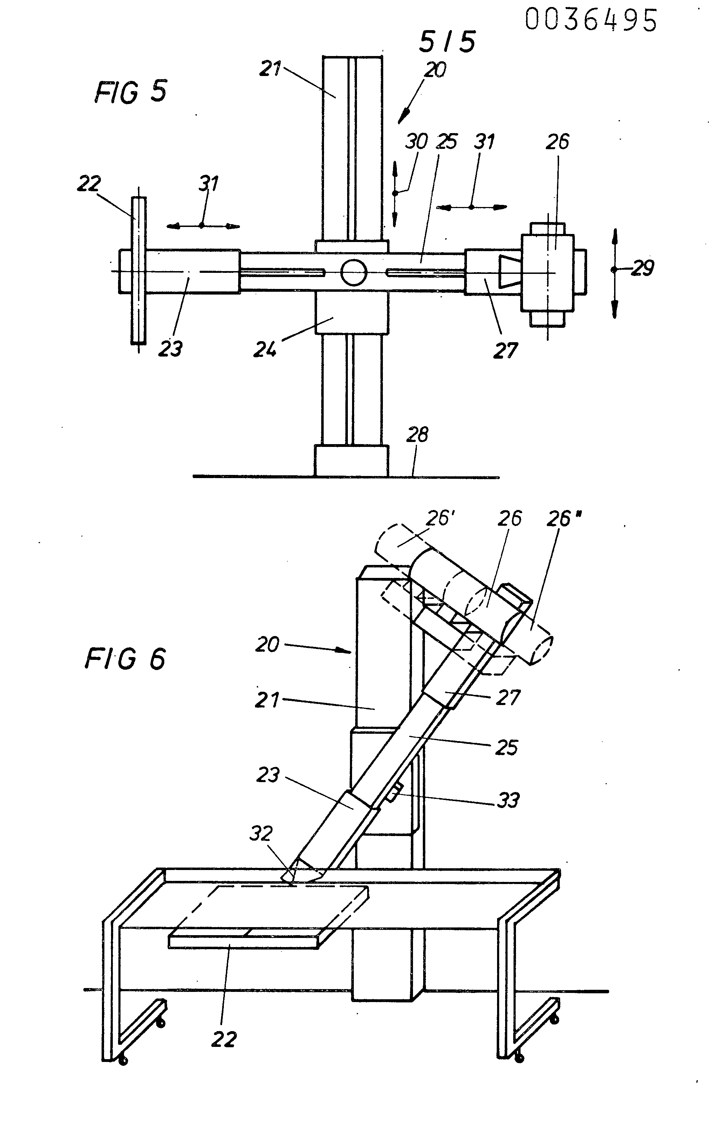

Das in Fig. 5 und 6 gezeigte Stativ 20 besteht aus einer vertikalen, auf einer Aufstellungsfläche 28 aufgestellten Wandsäule 21, an der in Pfeilrichtung 30 ein Vertikalwagen 24 verfahrbar angeordnet ist. Am Vertikalwagen 24 ist ein Drehzapfen angeordnet, an dem schwenkbar ein Querbalken 25 angeordnet ist. An einem freien Ende des Querbalkens 25 ist die Buckywand 22 an einem ersten Querwagen 23 in Pfeilrichtung 31 in Richtung der Längsachse des Querbalkens 25 verfahrbar angeordnet.

An dem anderen freien Ende des Querbalkens 25 ist die
Röntgenröhre 26 an einem zweiten Querwagen 27 angeordnet, der dann ebenfalls in den Pfeilrichtungen 31 in
Richtung der Längsachse am Querbalken 25 verschiebbar
ist.

Zusätzlich ist die Röntgenröhre 26 in senkrechter Richtung zur Längsachse des Querbalkens 25 in Pfeilrichtung
29 verfahrbar. Hierdurch kann die Aufnahmequalität der
eigenen Röntgenaufnahmen noch weiter verbessert werden.

Fig. 6 zeigt, dass der Querbalken 25 in beliebige Lagen
verschwenkbar ist und dass die Buckywand 22 über ein
Schwenklager 32 an dem ersten Querwagen 23 angeordnet
ist. Hierdurch kann die Buckywand 22 auch unter eine
Liege geschwenkt werden, um horizontal liegende Aufnahmen am Patienten durchführen zu können. Es ist ebenso das Schwenklager 33 des Querbalkens 25 gezeigt, und
es ist ferner sichtbar, dass die Röntgenröhre 26 in
die Stellungen 26', 26" am zweiten Querwagen 27 in
Pfeilrichtung 29 verfahrbar ist.

Patentansprüche

------------------------------------

1. Vorrichtung zur Steuerung der Strahlungsintensität
einer Röntgenanlage am Ort der Röntgenaufnahme durch
Änderung des Abstandes zwischen Röntgenröhre und Buckywand sowie Steuerung der Spannung und/oder des Milliampere-Sekunden-Produktes (MSP) der Röntgenröhre,
d a d u r c h   g e k e n n z e i c h n e t , dass
durch getrennt voneinander in einem Bedienungsfeld
(7, 8, 9) angeordnete Wahlschalter (1, 2, 3) folgende
Funktionen getrennt voneinander steuerbar sind:
a) Wahlschalter (1) für die verschiedenen zu durchleuchtenden Organe und Körperteile (Beschriftung (5))
steuert den Abstand zwischen Röntgenröhre (26) und
Buckywand (22) und gibt eine Mindest-Strahlungsintensität vor
b) Wahlschalter (2) ändert abhängig von der Lage des
Patienten im Strahlengang das Milliampere-Sekunden-
Produkt (MSP) und von der Grösse der Strahlungsintensität einer Laufrasterlade
c) Wahlschalter (3) als Sicherheitsschalter im Stromkreis der Aufnahmetaste (13) angeordnet, ändert das
MSP bei ABweichung von der körperlichen Norm des Patienten.

2. Vorrichtung nach Anspruch 1,   d a d u r c h
g e k e n n z e i c h n e t , dass dem Bedienungsfeld (7) mit dem Wahlschalter (1) für die verschiedenen zu durchleuchtenden Organe und Körperteile (Beschriftung (5)) ein Sichtfeld (15) mit Darstellung
des menschlichen Körpers zugeordnet ist und jedem der
Beschriftung (5) entsprechenden Körperteil im Bedienungsfeld (7) für den Wahlschalter (1) eine Kontroll-lampe (16a - e) an dem betreffenden Körperteil im
Sichtfeld (15) zugeordnet ist, (Fig. 1, 2).

3. Vorrichtung nach Anspruch 1 und 2,
d a d u r c h   g e k e n n z e i c h n e t , dass
der Wahlschalter (1) als mit separaten Tasten (4a - e)
ausgestattetes Bedienungsfeld (7) ausgebildet ist und
jedem zu durchleuchtenden Organ oder Körperteil eine
Taste (4a - e) mit Beschriftung (5) zugeordnet ist,
(Fig. 1).

4. Vorrichtung nach Anspruch 1 bis 3,
d a d u r c h   g e k e   n n z e i c h n e t , dass
der Wahlschalter (2) zur Wahl der Lage des Patienten
als mit separaten Tasten (4) ausgestattetes Bedienungsfeld (9) ausgebildet ist und jeder möglichen zu wählenden Lage eine Taste (4) mit Beschriftung (5) zugeordnet
ist, (Fig. 3).

5. Vorrichtung nach Anspruch 1 bis 4,
d a d u r c h   g e k e n n z e i c h n e t , dass
der Wahlschalter (3) zur Korrektur bei Abweichungen
von der körperlichen Norm als mit separaten Tasten (4)
ausgestattetes Bedienungsfeld (8) ausgebildet ist und
jedem möglichen Korrekturwert eine Taste (4) mit Beschriftung (5) zugeordnet ist, (Fig. 3).

6. Vorrichtung nach Anspruch 1 bis 5,
d a d u r c h   g e k e n n z e i c h n e t , dass
der Wahlschalter (1) zur Wahl der Durchleuchtung verschiedener Organe und Körperteile und der Wahlschalter (2) zur Wahl der Lage des Patienten die Antriebsmotoren des Stativs (20) der Röntgenanlage steuern,
(Fig. 5, 6).

7. Vorrichtung nach Anspruch 6, d a d u r c h
g e k e n n z e i c h n e t , dass das Stativ (20)
aus einer auf der Aufstellungsebene (28) angeordneten
vertikalen Wandsäule (21) besteht, an der in vertikaler
Richtung (30) verfahrbar ein Vertikalwagen (24) angeordnet ist, an dem schwenkbar ein zweiarmiger Querbalken (25) ansetzt, an dessen einem freien Ende die
Buckywand (22) verfahrbar an einem ersten Querwagen
(23) und an dessen anderem freien Ende die Röntgenröhre
(26) verfahrbar ain einem zweiten Querwagen (27) angeordnet sind, (Fig. 5).

8. Vorrichtung nach Anspruch 7, d a d u r c h
g e k e n n z e i c h n e t , dass der erste und zweite
Querwagen (23, 27) in Richtung der Längsachse des Querbalkens (25) verfahrbar sind, (Fig. 5).

9. Vorrichtung nach Anspruch 1 bis 8,
d a d u r c h g e k e n n z e i c h n e t , dass
die Röntgenröhre (26) senkrecht zur Längsachse des
Querbalkens (25) am Querwagen (27) verfahrbar angeordnet ist, (Fig. 5).

10. Vorrichtung nach Anspruch 6 bis 9,
d a d u r c h g e k e n n z e i c h n e t , dass
die elektrische Steuerung der Antriebsmotoren des
Stativs (20) mit einer Sicherheitsschaltung ausgerüstet
ist, die während der Verschwenkung bei drohender Beschädigung von beweglichen Teilen des Stativs (20)
an den Wänden des Aufstellungsraumes die Antriebsmotoren stillsetzt.

00364 95

115

□ Schädel —5
4a
□ Nasenbein —5
4b
5
□ Schädel, Teilaufnahme
4c

4d
□ Halswirbelsäule, HWS
4e
□ Brustwirbelsäule, BWS
4f
□ Lendenwirbelsäule, LWS

□ Becken — 7 —

□ Hüftgelenk

□ Oberschenkel

□ Kniegelenk

□ Kniescheibe

□ Unterschenkel

□ Sprunggelenk

□ Fuss

□ Fersenbein

□ Zehen

□ Schultergelenk
4
□ Schlüsselbein
4
□ Oberarm

□ Ellbogengelenk

□ Vorderarm

□ Handgelenk

□ Hand

□ Finger

□ Thorax

□ Rippenthorax
4
6 —○ □ Abdomen, Übersicht
4
6 —○ □ Gallenblase, - wege
4
6 —○ □ Nieren, Blase

○ □

○ □

} 1

FIG 1

0036495

215

FIG 2

16a
16 b
16c
16d
16e
16 f

15

0036495

FIG 3

FIG 4

0036495

Hauptschalter "ein" —— 14

- Lampe "wähle"
- Röhrenheiz.-Vorheizen —— 40
- Aufnahme gesperrt

Wahl der Exposition —— 41

Wahl der Korrektur    Wahl der Lage

Wahl der Lage    Wahl der Korrektur

gewählte Exp. i.o.    nein —— 42

ja

nein    gewählte Lage i.o. —— 43

ja

gewählte Korrektur i.o    nein —— 44

ja

autom. Einstellung —— 45
- der FFD
- des kV-Wertes
- des Röhrenheizwerts

Lampe "bereit" —— 12

Auslösung Vorbereitung

Röhrenheiz. Hochheizen —— 46
Drehanode ein
Buggy, wenn prog., ein
Spannungsregler aus
Expositioneing. gesperrt

—— 47

Spannungsregler i.o.    nein
Heizkreisregler i.o.         keine Aufnahme mögl.
Anode hochgelaufen
Buckyrückmeldung i.o

ja

ungewollte Auslös.
e. Aufnahme w. Defekts

48 —— Auslösung Aufnahme

49 —— Zeitgeber    Bestr. Zeit ≠ prog Zeit    Bestr. Zeit ≥ 8 s

Bestr. Zeit =
prog. Zeit

Ende Aufnahme

Auslösung Sicherheitskreis und aut. Ausserbetriebsetzung des Gerätes

Hauptschalter "aus"

0036495

FIG 5

5 / 5

FIG 6

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 2 220 444 (KOCH & STERZEL KG)<br><br>* Seite 2, Zeile 28 bis Seite 3, Zeile 8; Seite 4, Zeile 22 bis Seite 5, Zeile 10; Abbildung * | 1-4,6 |
| | --- | |
| | FR - A - 2 246 256 (SIEMENS AKTIENGESELLSCHAFT)<br><br>* Seite 1, Zeilen 1-6 und 18-39; Seite 2, Zeilen 16-28; Seite 2, Zeile 40 bis Seite 3, Zeile 8; Seite 7, Zeilen 1-27; Abbildung 1 *<br><br>& DE - A - 2 350 141 | 1-5 |
| | -- | |
| | FR - A - 2 412 223 (SIEMENS AKTIENGESELLSCHAFT)<br><br>* Seite 1, Zeilen 1-39; Seite 2, Zeilen 33-40; Seite 3, Zeile 10 bis Seite 4, Zeile 3;; Seite 8, Zeilen 6-10; Abbildung 1 *<br><br>& DE - A- 2 755 736 | 1-5 |
| | -- | |
| | US - A - 4 080 536 (SIEMENS AKTIENGESELLSCHAFT<br><br>* Spalte 1, Zeile 37 bis Spalte 2, Zeile 9; Spalte 4, Zeile 45 bis Spalte 5, Zeile 4; Abbildungen 1,4 *<br><br>DE - A - 2 523 887 | 1-4 |
| | -- | |
| | DE - A - 2 406 424 (COMPAGNIE GENERALE DE RADIOLOGIE) | 1-4 |

./.

**KLASSIFIKATION DER ANMELDUNG (Int. Cl.)**

H 05 G 1/46
A 61 B 6/00

**RECHERCHIERTE SACHGEBIETE (Int. Cl.)**

H 05 G 1/02
1/26
1/30
1/32
1/34
1/36
1/38
1/46
1/54
A 61 B 6/00
6/10

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angefuhrtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prufer |
|---|---|---|
| Den Haag | 07-07-1981 | HORAK |

EPA form 1503.1   06.78

Europäisches
Patentamt

**EUROPÄISCHER RECHERCHENBERICHT**

0036495
Nummer der Anmeldung

EP 81 10 1330

-2-

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl. |
|---|---|---|---|
| | * Seite 1, Zeile 1 bis Seite 2, Zeile 7; Abbildung 1 * <br> & DE - A - 2 747 310 <br><br> -- <br><br> FR - A - 2 132 403 (SIEMENS AG) <br> * Seite 2, Zeile 30 bis Seite 3, Zeile 26; Seite 6, Zeilen 2-13; Abbildungen 1,2 * <br> & DE - A - 2 116 705 <br><br> -- | <br><br><br><br><br><br> 1,6 | |
| A | DE - C - 976 085 (KOCH & STERZEL KG) <br> * Seite 1, Zeile 1 bis Seite 2, Zeile 85 * <br><br> -- | 1-5 | RECHERCHIERTE SACHGEBIETE (Int. Cl. |
| A | GB - A - 2 026 206 (N.V. PHILIPS' GLOEILAMPENFABRIEKEN) <br> * Seite 1, Zeilen 6-10, 17-22 und 52-59; Seite 6, Zeilen 26-43; Abbildung 1 * <br> & DE - A - 2 831 058 <br><br> -- | 1,6-9 | |
| A | DE - A - 1 566 157 (SIEMENS AG) <br> * Seite 8, Zeilen 1-23 * <br><br> ----- | 1,6, 10 | |